# FASCICULE DE BREVET EUROPEEN

(11) **EP 0 565 599 B1**
(45) Date de publication et mention de la délivrance du brevet: **14.12.1994**
(21) Numéro de dépôt: 92903068.2
(22) Date de dépôt: 03.01.1992
(51) Int. Cl.: G01N 21/64, A61N 5/06, A61B 5/00

(54) **DISPOSITIF DE MESURE DU pH D'UNE CIBLE, PROCEDE D'UTILISATION DUDIT DISPOSITIF ET SES APPLICATIONS**
VORRICHTUNG ZUM MESSEN DES PH-WERTES EINER PROBE, METHODE ZUR ANWENDUNG EINER SOLCHEN VORRICHTUNG UND ANWENDUNGEN
DEVICE FOR MEASURING THE pH OF A TARGET, METHOD FOR USING SUCH DEVICE AND APPLICATION THEREOF

(30) Priorité: 04.01.1991 FR 9100064
(43) Date de publication de la demande: 20.10.1993
(73) Titulaire: INSTITUT NATIONAL DE LA SANTE ET DE LA RECHERCHE MEDICALE (INSERM), 75654 Paris Cédex 13 (FR)
(72) Inventeur: MORDON, Serge, F-59491 Villeneuve-d'Ascq (FR); MAUNOURY, Vincent, F-59130 Lambersart (FR); DEVOISSELLE, Jean-Marie Le Fontenelle G4, F-34090 Montpellier (FR)
(74) Mandataire: Ores, Irène
(86) Numéro de dépôt international: FR9200006
(87) Numéro de publication internationale: WO9212412

(56) Documents cités:
- WO-A-84/04665
- WO-A-90/10219
- GB-A- 2 126 717
- US-A- 4 768 513
- US-A- 4 973 848
- MEDICAL AND BIOLOGICAL ENGINEERING & COMPUTING, vol. 25, no. 6, November 1987, pages 597-604, Stevenage, Herts., GB ; M.J. MARTIN et al. : "Fibre-optics and optical sensors in medicine".
- D. LANSING TAYLOR et al. : "Methods in Cell Biology", vol. 30, part B, pages 127-156, Academic Press, pages 131-140.
- IEEE TRANSACTIONS ON BIOMEDICAL ENGINEERING, vol. BME-33, No. 2, February 1986, pages 117-132 ; J.L. GEHRICH et al. : "Optical Fluorescence and Its Application to an Intravascular Blood Gas Monitoring System".
- MEDICAL PHYSICS, vol. 11, no. 4, July/August 1984, pages 516-520, New York, US ; A.E. PROFIO et al. : "Fluorometer for endoscopic diagnosis of tumors".

## Description

La présente invention a pour objet un dispositif de mesure du pH d'une cible appropriée, notamment une tumeur, un procédé d'utilisation dudit dispositif ainsi que ses applications, notamment au contrôle du traitement des tumeurs par hyperthermie.

Le dépistage précoce des tumeurs est essentiel pour l'amélioration de leur pronostic ; cependant, les tumeurs d'organes ou de tissus sont souvent difficiles à détecter et, dans le cas du cancer de l'oesophage, par exemple, l'avènement de la fibroscopie oesophagienne, n'a pas amélioré la précocité de son diagnostic.

D'autres méthodes de diagnostic précoces ont été proposées et font appel à plusieurs techniques :
1 - fixation d'un agent de contraste ou d'un agent colorant et diagnostic par imagerie ; l'agent colorant peut soit présenter une grande affinité pour les acides nucléiques (bleu de toluidine, par exemple), soit au contraire ne pas présenter de fixation tumorale (lugol, par exemple, qui ne réagit qu'avec le glycogène de l'épithélium malpighien différencié), soit enfin par l'emploi de photosensibilisateurs présentant un pic de fluorescence lors d'une excitation lumineuse adaptée (photodiagnostic). Ces techniques impliquent l'obtention d'un gradient de fluorescence entre tissu sain et tissu tumoral, qui nécessite la fixation élective ou la rétention particulière du marqueur par la tumeur ; or, celles-ci sont dépendantes de nombreux facteurs et notamment de la vascularisation, de la nécrose et de la capacité phagocytaire de la tumeur.
2 - l'utilisation d'anticorps monoclonaux ou la vectorisation du marqueur par des liposomes ; cette technique, utilisée seule, a l'inconvénient de nécessiter des réactifs spécifiques et coûteux.
3 - l'étude spectrale de la fluorescence émise. Cette dernière méthode, qui ne nécessite pas l'utilisation de réactifs spécifiques, peut éviter les inconvénients précités.

On peut citer, comme méthodes de diagnostic utilisant l'étude spectrale de la fluorescence émise :
- le procédé de diagnostic proposé par S. ANDERSSON-ENGELS et al. (Lasers in Medical Science, 1988, 4, 171-181), qui décrit la localisation et la détection de plaques d'athérome par la mesure de l'autofluorescence induite par un laser comme source d'excitation lumineuse ;
- le diagnostic des tumeurs par analyse de la fluorescence, qui a notamment été décrit dans :

. l'article au nom de R.R. ALFANO et al., paru dans J. Quantum Electronics, 1984, vol. OE-20, 12, 1507-1511, qui décrit la mesure de l'autofluorescence induite par une source laser, tant sur les tissus cancéreux que sur les tissus sains et qui montre que les profils spectraux des tissus cancéreux sont différents de ceux des tissus sains.
. et les méthodes par fluorescence provoquée, comme précisé dans l'article au nom de A.E. PROFIO et al., paru dans Med. Phys., 1984, 11, 4, 516-520, qui décrit un fluoromètre pour le diagnostic endoscopique de tumeurs. Plus précisément, un dérivé fluorescent de l'hématoporphyrine est injecté, puis on caractérise la tumeur en détectant la fluorescence émise ; la source d'excitation est une lumière violette, acheminée à travers une fibre optique, jusqu'à l'endoscope, alors que la fluorescence émise ainsi que la lumière violette réfléchie sont collectées par une autre fibre optique. La fluorescence en lumière rouge et en lumière violette sont séparées à l'aide d'un miroir dichroïque et d'un filtre, et détectées à l'aide de photomultiplicateurs. Cette méthode a l'inconvénient de localiser difficilement les petites tumeurs, d'être absolument dépendante des conditions de mesure et le gradient de concentration entre tissus sain et tumoral est faible avec, de plus, un risque de phototoxicité cutanée.

- Une autre méthode par fluorescence provoquée est précisée dans le Brevet GB n° 2 126 717 A, qui décrit un dispositif pour le diagnostic et le traitement des cancers.

Ce dispositif comprend au moins une source laser pulsée, un moyen de détection d'images, un moyen de contrôle et un endoscope. L'extrémité de l'endoscope est en face de l'endroit où un produit photosensible ayant une affinité pour les cancers a été absorbé, de telle sorte que le diagnostic du cancer peut être réalisé en exposant l'organisme affecté à la source laser. Cette méthode présente l'inconvénient d'être également dépendante des conditions de mesure.

Les méthodes de diagnostic de tumeurs, par fluorescence, proposées dans l'Art antérieur, ont, de plus, l'inconvénient majeur d'être des techniques absolues, qui entraînent de nombreux faux positifs ou faux négatifs, car elles sont dépendantes des conditions de mesure (position des fibres de recueil, par exemple ou structure du tissu à étudier).

Un certain nombre de documents décrivent des optrodes (optic electrodes) pour la mesure du pH (Medical & Biological Engineering & Computing, 1987, 25, 5, 597-604 ; IEEE Transactions on Biomedical Engineering, 1986, BME-33, 117-132) ; cependant, de telles optrodes présentent l'inconvénient majeur de ne pas pouvoir être utilisées dans un système d'imagerie, dans la mesure où elles ne permettent qu'une mesure ponctuelle, au point de contact avec le tissu concerné.

La présente invention s'est en conséquence donné pour but de pourvoir à un dispositif et à un procédé qui permettent de résoudre le problème de la détection d'une tumeur difficilement détectable par les méthodes de l'Art antérieur ou dont les résultats sont difficilement interprétables, et également de résoudre le problème de la dépendance vis-à-vis des conditions de mesure, rencontrée dans les systèmes proposés dans l'Art antérieur (A.E. PROFIO et al.) ; en effet, la résolution de ce problème est cruciale pour l'obtention de résultats qui sont fiables et permettent d'éviter les faux positifs et les faux négatifs.

Les Inventeurs ont, pour ce faire, utilisé les mesures du métabolisme cellulaire et plus particulièrement la mesure du pH intracellulaire à l'aide de marqueurs fluorescents à spectres dépendants du pH, comme cela a notamment été précisé dans J.A. THOMAS et al., (Biochem., 1979, 18, 2210-2218), qui décrit les caractéristiques spectrales de la fluorescéine et de la 6-carboxyfluorescéine (6-CF), qui sont dépendantes du pH, dans le but de détecter la présence éventuelle d'une tumeur. L'absorbance à 490 nm (pic) en référence à l'absorbance à 465 nm (point isosbestique, c'est-à-dire indépendant du pH) montre que l'absorbance est plus importante à pH 7,8 qu'à pH 6,25.

La présente invention a pour objet un dispositif de mesure du pH d'une cible, du type comprenant une source lumineuse convenable pour la sélection d'au moins deux longueurs d'onde d'excitation de la fluorescence d'un marqueur fluorescent fixé sur ladite cible et dont le spectre d'émission de fluorescence est dépendant du pH et un moyen de détection et de lecture de la fluorescence émise,
lequel dispositif est caractérisé
* en ce qu'il comprend, pour la mesure dudit pH sans contact direct avec ladite cible :
   - au moins un moyen de transmission de ladite source lumineuse à la cible,
   - au moins un moyen de recueil de la fluorescence émise ;
   - un système de calcul du pH de la cible à partir du rapport des signaux de fluorescence émise, obtenus successivement au moins auxdites deux longueurs d'ondes d'excitation, et
* en ce que la source lumineuse est associée à un moyen de bascule d'une longueur d'onde d'excitation à l'autre.

Selon un mode de réalisation avantageux dudit dispositif, le moyen de transmission de la source lumineuse comprend au moins une fibre optique.

Selon un autre mode de réalisation avantageux dudit dispositif, le moyen de recueil de fluorescence comprend au moins une fibre optique.

On entend, par fibre optique, au sens de la présente invention, toute fibre en matière diélectrique, destinée à guider des ondes électromagnétiques, visibles ou infrarouges, par exemple.

Conformément à l'invention ledit moyen de transmission et/ou ledit moyen de recueil sont associés à un endoscope, couplé à un intensificateur d'image, lui-même relié à une caméra vidéo.

On entend, par endoscope, au sens de la présente invention, aussi bien les endoscopes classiques, c'est-à-dire les appareils destinés à éclairer et à rendre visible l'intérieur d'une cavité du corps humain que les fibroscopes, c'est-à-dire les endoscopes souples formés d'un faisceau de fibres optiques extrêmement fines.

Selon une disposition avantageuse de ce dernier mode de réalisation, ledit moyen de transmission et/ou ledit moyen de recueil sont inclus dans l'endoscope.

Un tel dispositif permet d'obtenir à la fois une image de fluorescence et une image visible de ladite cible.

Le moyen de mesure des signaux de fluorescence émis par la cible marquée, est avantageusement un spectrophotomètre, et comprend notamment au moins un filtre optique, au moins un photomultiplicateur et/ou au moins un photodétecteur de conversion desdits signaux lumineux, et un moyen d'acheminement des signaux électriques correspondants vers le système de calcul du pH.

Les marqueurs fluorescents sensibles au pH sont plus particulièrement décrits dans l'article paru au nom de R.Y. TSIEN (Methods in cell Biology, 1989, 30, 127-156), dans lequel il est notamment précisé que les marqueurs fluorescents suivants : la fluorescéine, la fluorescéine conjuguée au dextran ou à une autre molécule inerte (DF), la 5 et/ou 6 carboxyfluorescéine (CF), le 2′,7′-bis(carboxyéthyl)-5 et/ou 6-carboxyfluorescéine (BCECF) et leurs esters, la pyramine (8-hydroxypyrène-1,3,6-trisulfonate), le 4-méthylumbelliferone ou 4-méthyl-7-hydroxycoumarine (4-MU), le 3,6 dicyanohydroquinone (DHPN), le SNARF-1 et le SNAF-2 (respectivement le semi-naphtorhodofluor et la semi-naphtofluorescéine), présentent une paire de longueurs d'onde dont le rapport d'excitation augmente avec le pH.

Ces marqueurs permettent notamment de mesurer le pH du cytosol.

Conformément à l'invention, le marqueur fluorescent est avantageusement associé à des liposomes et/ou des anticorps monoclonaux appropriés.

Selon un autre mode de réalisation du dispositif conforme à l'invention, le système de calcul du pH comprend avantageusement un moyen de calcul du rapport des signaux de fluorescence émise, obtenus successivement au moins auxdites deux longueurs d'ondes d'excitation, et un moyen de lecture du pH correspondant au rapport obtenu, sur une courbe d'étalonnage dudit marqueur, en fonction du pH.

Selon une disposition avantageuse de ce mode de réalisation, ledit système de calcul comprend également un système de contrôle du moyen de bascule.

Un tel système est notamment représenté par un microordinateur approprié, qui permet à la fois d'obtenir le pH en fonction des rapports de signaux de fluorescence émise et de contrôler le moyen de bascule d'une longueur d'onde d'excitation à une autre.

Lorsque la cible est une cellule, un tissu ou un organe, le dispositif conforme à l'invention présente les avantages suivants :
- la mesure du pH est réalisée, *in situ*, sans contact direct avec lesdits cellule, tissu ou organe ;
- cette mesure de pH permet la détection d'une tumeur, car les cellules tumorales présentent un pH plus acide que les cellules normales, dans la mesure où les cellules tumorales sont généralement anoxiques et ont donc recours à la glycolyse anaérobie, avec pour conséquence la production d'acide lactique ;
- cette mesure du pH est fiable, car elle utilise un rapport d'intensités de fluorescence à deux longueurs d'ondes différentes, ce qui évite la dépendance vis-à-vis des paramètres de mesure, (structure de la cible, angle entre la fibre de recueil et la fluorescence émise par la cible, notamment), souvent importante dans les mesures de fluorescence et ce qui permet d'obtenir des signaux de bonne qualité et éventuellement de les amplifier.

La présente invention a également pour objet un dispositif de contrôle de l'hyperthermie dans le traitement des tumeurs par hyperthermie, caractérisé en ce qu'il comprend un dispositif de mesure de pH conforme à l'invention, associé à un moyen de chauffage approprié de ladite tumeur.

Ledit moyen de chauffage est avantageusement choisi parmi l'un quelconque des moyens suivants : source laser, micro-ondes, ultra-sons, radiofréquence ou circuit d'eau chaude.

Conformément à l'invention, ledit moyen de chauffage est notamment associé à un moyen de focalisation de la chaleur produite, vers la tumeur, notamment une sonde, un cathéter ou une antenne extérieure.

La présente invention a également pour objet un procédé d'utilisation du dispositif conforme à l'invention, permettant d'obtenir les informations caractéristiques de l'évolution dans le temps du pH d'une cible appropriée, caractérisé en ce qu'il comprend :
- la mise en contact de la cible à analyser avec un marqueur fluorescent, présentant, au moins deux pics d'excitation et un pic d'émission et dont le spectre d'émission est dépendant du pH ;
- l'excitation de la cible ainsi traitée, successivement auxdites longueurs d'onde d'excitation dudit marqueur fluorescent ;
- la mesure de la fluorescence émise par ladite cible à analyser, successivement auxdites longueurs d'onde d'excitation ; et
- le calcul du pH de ladite cible à analyser à partir du rapport des signaux de fluorescence émis, obtenus successivement au moins auxdites deux longueurs d'onde d'excitation, par la lecture du pH correspondant au rapport obtenu, sur une courbe d'étalonnage dudit marqueur, en fonction du pH.

Selon un mode de mise en oeuvre avantageux dudit procédé, préalablement à la mise en contact de la cible à analyser avec le marqueur fluorescent, ladite cible est mise en contact avec un sucre, notamment du glucose.

La mise en contact de la cible avec le sucre rend le procédé encore plus sensible, dans la mesure où il permet de diminuer encore le pH des tissus tumoraux par rapport aux tissus sains et donc d'augmenter ainsi la différence entre tissu tumoral et tissu sain.

Selon un mode de mise en oeuvre avantageux dudit procédé, le marqueur fluorescent est notamment choisi dans le groupe qui comprend la fluorescéine, la fluorescéine conjuguée au dextran ou à une autre molécule inerte (DF), la 5 et/ou 6 carboxyfluorescéine (CF), le 2′,7′-bis(carboxyéthyl)-5 et/ou 6-carboxyfluorescéine (BCECF) et leurs esters, la pyramine (8-hydroxypyrène-1,3,6-trisulfonate), le 4-méthylumbelliferone ou 4-méthyl-7-hydroxycoumarine (4-MU), le 3,6 dicyanohydroquinone (DHPN), le SNARF-1 et le SNAF-2 (respectivement le semi-naphtorhodofluor et la semi-naphtofluorescéine).

Selon une disposition avantageuse de ce mode de mise en oeuvre, ledit marqueur fluorescent est associé à des liposomes et/ou des anticorps monoclonaux appropriés.

Les dispositifs et le procédé conformes à l'invention s'appliquent de manière particulièrement avantageuse à la mesure du pH de tumeurs, notamment accessibles uniquement par voie endoscopique et permettent de suivre le pH de la tumeur en différents points et l'évolution du traitement par hyperthermie desdites tumeurs, ce qui rend possible le calcul de la dose thermique utile, en n'importe quel point de la tumeur ; en effet, la dose thermique à appliquer est différente selon le pH.

Le dispositif conforme à l'invention permet également le contrôle en temps réel de la dose thermique à appliquer.

Outre les dispositions qui précèdent, l'invention comprend encore d'autres dispositions, qui ressortiront de la description qui va suivre, qui se réfère à des exemples de mise en oeuvre du procédé conforme à l'invention et à une description détaillée du dispositif selon l'invention, avec référence aux dessins annexés, dans lesquels :
- les figures 1 et 2 représentent schématiquement deux mode de réalisation d'un dispositif de mesure du pH conforme à l'invention ;
- la figure 3 représente le profil cinétique de fluorescence de deux cibles saines (S₁ et S₂) ;
- la figure 4 représente l'effet du 5,6-CF sur les valeurs du rapport d'intensités de fluorescence d'émission de deux zones saines de la peau, calculé à partir des intensités de fluorescence obtenues à 490 et 465 nm ;
- la figure 5 représente le profil cinétique de fluorescence de tissus normaux et tumoraux après une injection de 250 µl de 5,6-CF à 10⁻³ M ;
- la figure 6 représente l'effet d'un tissu sain ou d'un tissu tumoral sur les valeurs des rapports d'intensités de fluorescence, calculés à partir des intensités obtenues à 490 et 465 nm ;
- la figure 7 représente l'évolution du spectre d'émission de fluorescence du 5,6-CF en fonction du pH ;
- la figure 8 représente la courbe de calibration de la 6 CF en fonction du pH ;
- la figure 9 montre l'influence du pH sur un traitement par hyperthermie.

Il doit être bien entendu, toutefois, que ces exemples sont donnés uniquement à titre d'illustration de l'objet de l'invention, dont ils ne constituent en aucune manière une limitation.

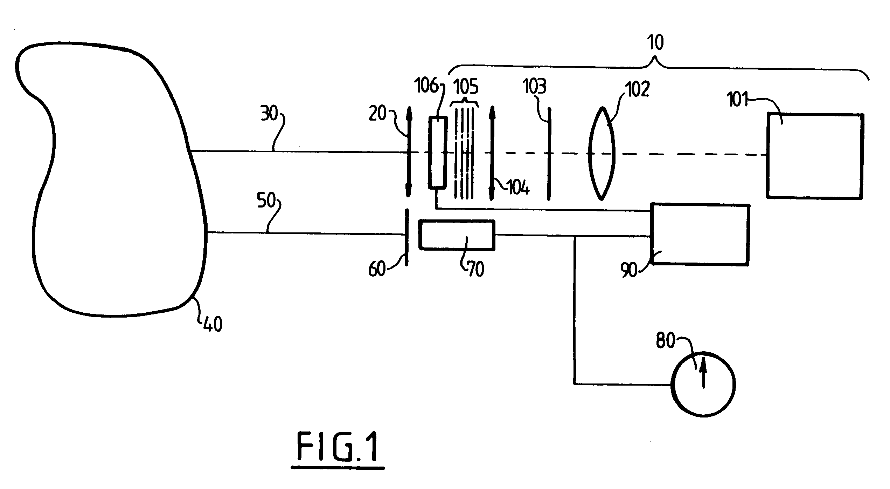

La figure 1 représente une vue schématique d'un dispositif de mesure de pH conforme à l'invention, comprenant :
- une source d'excitation 10, qui dans la réalisation représentée, et ce de manière non limitative, comporte une lampe à arc USHIO 155 XENON (USHIO, JAPON) 101, un miroir sphérique de rayon de courbure 3 cm 102, un condenseur 66061 ORIEL 103 (ORIEL, USA), une lentille convergente de diamètre 7,5 cm et de distance focale 200 mm 104, et des filtres interférentiels 105 centrés sur 450 nm, 465 nm, 490 nm et 500 nm, permettant de sélectionner les longueurs d'onde désirées ; un moyen de bascule 106 ou commutateur permet de basculer d'une longueur d'onde d'excitation à une autre ; une dernière lentille 20, de diamètre 22,4 mm permet de focaliser la lumière sur le coeur d'une fibre 600 µm de transmission 30 (O.N. = 0,48). La puissance délivrée est de 3 mW sur une bande de 10 nm. La fibre de transmission est au contact de la cible 40, notamment un tissu, dont le pH est à analyser *in situ* ;
- une fibre de recueil 50, d'un diamètre de 600 µm, est connectée à un monochromateur MC1-03 (OPTOMETRICS, USA) 60 relié, à un photomultiplicateur 70 (MINI-CHROM ; bande 300 nm à 800 nm) ;
- la lecture du signal s'effectue, dans la réalisation représentée sur un voltmètre DIPITOL 80 ;
- le système de calcul est représenté par un microordinateur 90, auquel sont reliés le système de recueil (fibre de recueil 50, monochromateur 60 et photomultiplicateur 70) et le moyen de bascule 106.

Le système de calcul permet le calcul du rapport des signaux de fluorescence émise obtenus à deux longueurs d'excitation appropriées au marqueur utilisé, puis le calcul du pH de la cible 40 correspondant au rapport obtenu, sur une courbe d'étalonnage dudit marqueur en fonction du pH.

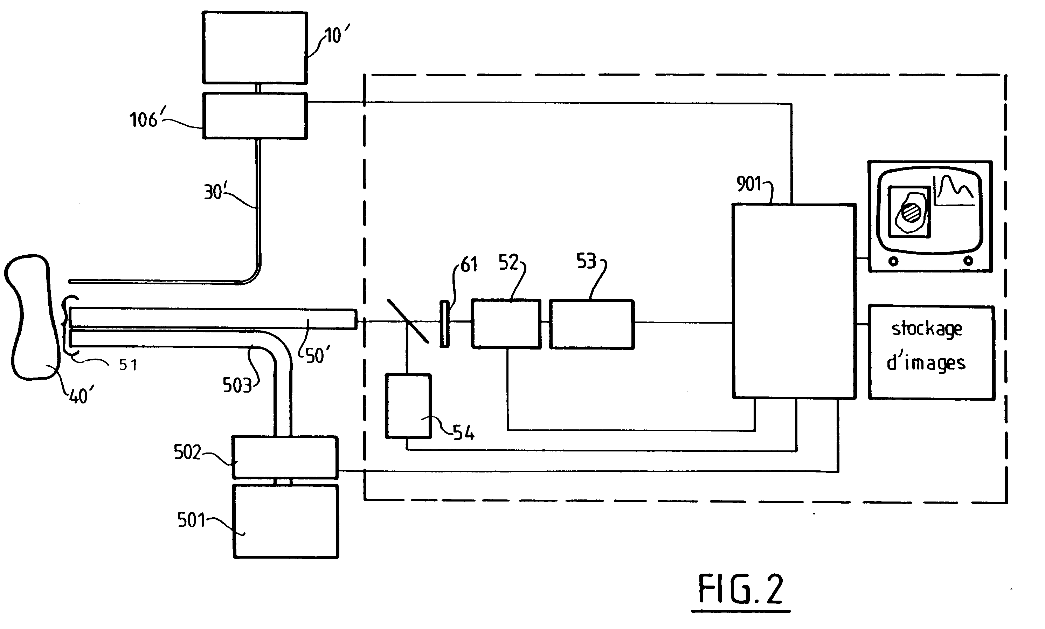

La figure 2 représente une vue schématique d'un dispositif de mesure de pH conforme à l'invention, dans lequel la fibre de recueil 50′ est incluse dans un endoscope 51 qui permet ainsi d'obtenir à la fois une image de fluorescence et une image dans le visible.

Le dispositif représenté à la figure 2 comprend :
. une source d'excitation de fluorescence 10′, dont les caractéristiques peuvent être identiques à celle de la source décrite à l'exemple 1, qui est associée à un commutateur de longueur d'onde d'excitation 106′, qui peut avantageusement être contrôlé par un ordinateur 901 ; le commutateur 106′ est relié à la cible 40′ par l'intermédiaire d'une fibre de transmission 30′ de même type que celle de l'exemple 1 ;
. un endoscope 51, qui comprend une source lumineuse 501, un obturateur 502 et un faisceau de fibres appropriées 503 ;
. une fibre de recueil 50′, qui est incluse dans un endoscope 51, est associée à un filtre 61 de sélection de la longueur d'onde d'émission (515 nm, lorsque le marqueur est la 6-CF, par exemple) ou à un monochromateur; elle est couplée d'une part à un intensificateur d'image 52, lui-même relié à une caméra 53 et d'autre part à une caméra vidéo 54, qui permet l'obtention d'une image de la cible.

Les signaux de fluorescence (image de fluorescence) et l'image dans le visible sont dirigées vers le microordinateur 901.

Le système de recueil (fibre de recueil 50′, filtre 61 ou monochromateur, intensificateur d'image 52, caméra 53), l'endoscope 51, la caméra vidéo 54 et l'ordinateur 901 constituent un système d'imagerie qui permet à la fois le stockage d'images et de spectres de fluorescence.

Les dispositifs conformes à l'invention, permettent la mesure du pH d'une cible.

On procède comme suit : la mesure est réalisée sur des souris CDF porteuses d'une leucémie lymphoïde P388.

La tumeur est greffée en sous-cutané sur un côté de la souris.

L'évolution spontanée de la tumeur est reproductible : une tumeur d'un diamètre de 20 mm est obtenue 12 jours après la greffe.

Une injection intrapéritonéale de 250 µl de 5,6-CF dans un tampon NaCl 0,9 % à une concentration de 10⁻³ M, 5.10⁻³ M ou 10⁻⁴ M (5 mg/kg, 2,5 mg/kg ou 0,5 mg/kg), est réalisée au jour 12.

Avant de mesurer la fluorescence et de calculer le pH de la tumeur, la zone tumorale et un tissu sain correspondant sont rasés.

L'injection de 5,6 CF étant considérée comme le temps zéro, les intensités de fluorescence sont mesurées aux temps -3 minutes, +3 minutes, puis toutes les 10 minutes, pendant 1 heure.

Des essais contrôles sont réalisés sur la peau normale et sur des tumeurs, n'ayant pas été mises en contact avec le marqueur fluorescent.

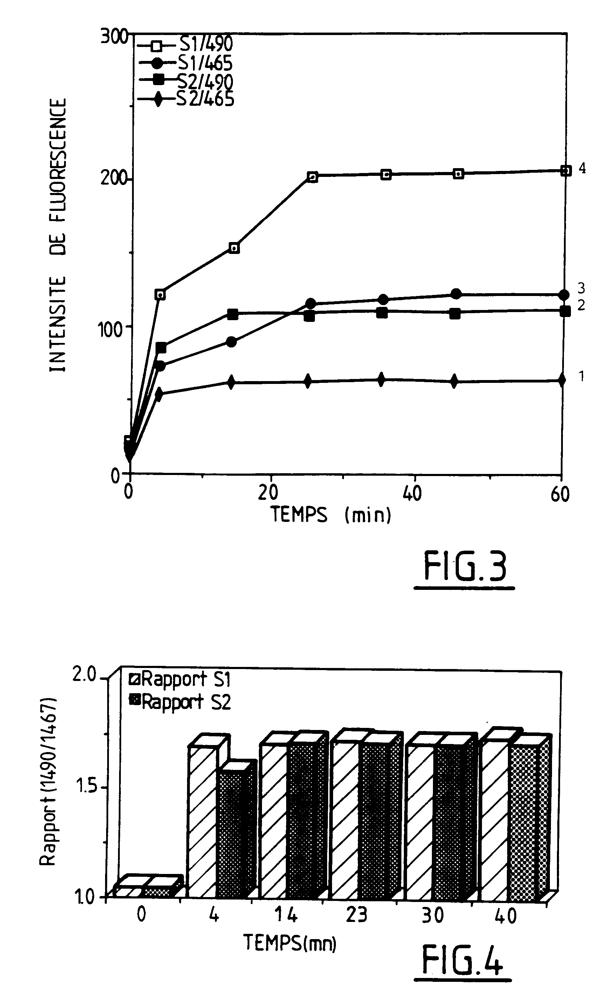

La figure 3, qui comporte en abscisse, le temps en minutes et en ordonnées, les intensités de fluorescence, montre les différents profils cinétiques entre deux zones de tissus sains (S1 et S2). Les intensités de fluorescence maximales peuvent être relativement différentes ; les intensités maximales sont atteintes en 5 à 30 minutes, mais sont différentes pour chaque tissu. Les différences dans les profils cinétiques peuvent être dues notamment à des perturbations dans les conditions de détection dans les deux zones. Les courbes 1 et 2 montrent les profils cinétiques du tissu S2, respectivement à 465 et 490 nm et les courbes 3 et 4 montrent les profils cinétiques du tissu S1, respectivement à 465 et à 490 nm.

La figure 4 montre que quelles que soient les intensités de fluorescence dans les deux zones, les différences n'affectent pas les valeurs des rapports, qui restent constants et sont quasiment identiques pour les deux zones S1 et S2 entre 15 min et 50 min après l'injection.

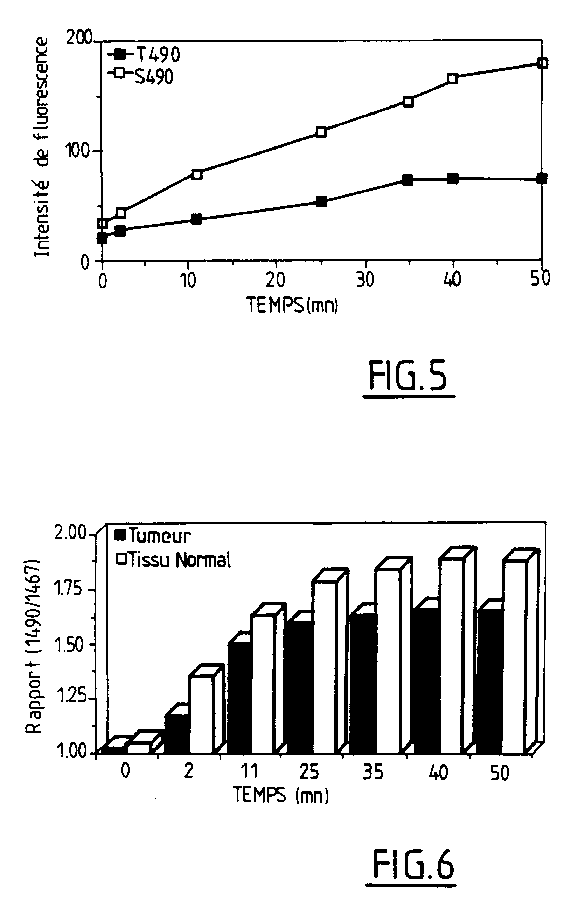

La figure 5 montre les intensités de fluorescences obtenues en fonction du temps, sur un tissu sain (-□-) et sur un tissu tumoral (-■-). Cette figure donne les profils cinétiques obtenus après injection de CF à 10⁻³ M. La fluorescence des tissus normaux est supérieure à celle des tissus tumoraux.

La figure 6 montre les valeurs des rapports I490/I465 et montrent qu'il existe bien une différence significative entre les tissus sains et les tissus tumoraux.

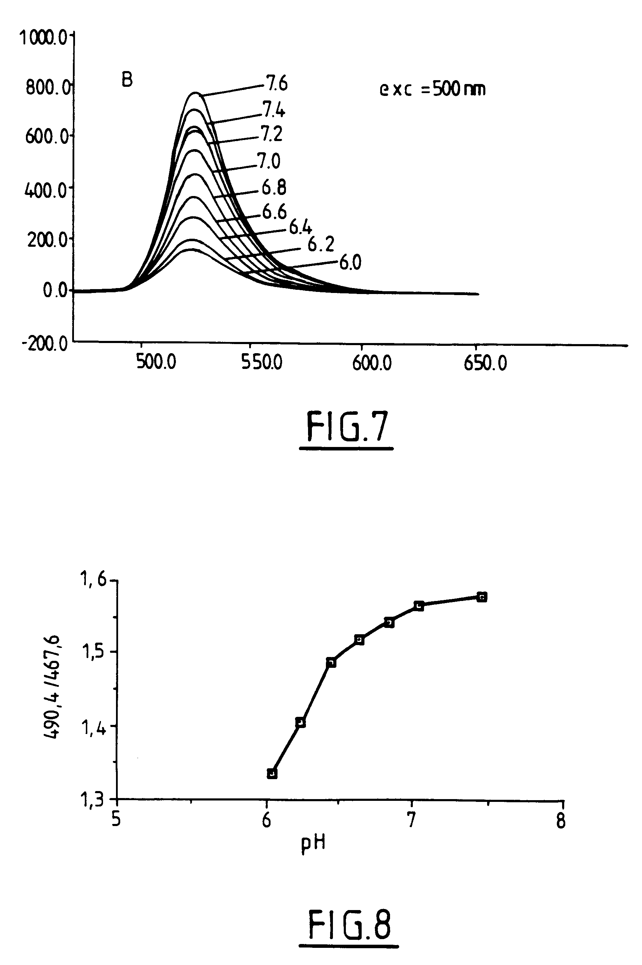

Les résultats montrent que la pénétration de la lumière d'excitation est suffisante pour détecter les variations spectrales de fluorescence par la méthode des rapports et permettent d'obtenir la valeur du pH au niveau de la tumeur ; en effet, la figure 7 montre l'évolution du spectre d'émission de fluorescence de la carboxyfluorescéine en fonction du pH et la figure 8 montre la courbe d'étalonnage de la 5,6 CF en fonction du pH.

Le procédé conforme à l'invention permet également d'évaluer la dose thermique (durée et temps) nécessaire pour détruire des cellules tumorales en fonction du pH desdites cellules.

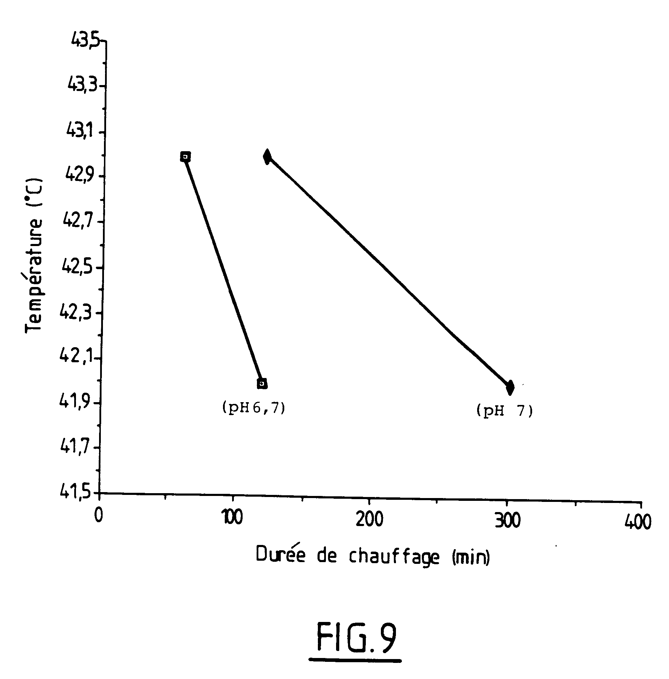

La figure 9 montre l'influence du pH sur la résistance de cultures de cellules soumises à une hyperthermie.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention comme revendiquée.

## Revendications

1. Dispositif de mesure du pH d'une cible, du type comprenant une source lumineuse (101) convenable pour la sélection d'au moins deux longueurs d'onde d'excitation de la fluorescence d'un marqueur fluorescent fixé sur ladite cible et dont le spectre d'émission de fluorescence est dépendant du pH et un moyen de détection et de lecture de la fluorescence émise (60, 70, 80 ; 61,52,53),
lequel dispositif est caractérisé
* en ce qu'il comprend, pour la mesure dudit pH sans contact direct avec ladite cible :
- au moins un moyen de transmission (30, 30′) de ladite source lumineuse à la cible (40, 40′),
- au moins un moyen de recueil (50, 50′) de la fluorescence émise ;
- un système de calcul du pH de la cible à partir du rapport des signaux de fluorescence émise, obtenus successivement au moins auxdites deux longueurs d'ondes d'excitation (90, 901), et
* en ce que la source lumineuse est associée à un moyen de bascule (106, 106′) d'une longueur d'onde d'excitation à l'autre.

2. Dispositif selon la revendication 1; caractérisé en ce que le moyen de transmission de la source lumineuse comprend au moins une fibre optique.

3. Dispositif selon la revendication 1 ou la revendication 2, caractérisé en ce que le moyen de recueil de fluorescence comprend au moins une fibre optique.

4. Dispositif selon l'une quelconque des revendications 1 à 3, caractérisé en ce que ledit moyen de transmission et/ou ledit moyen de recueil sont associés à un endoscope (51), couplé à un intensificateur d'image (52), lui-même relié à une caméra vidéo (53).

5. Dispositif selon la revendication 4, caractérisé en ce que ledit moyen de transmission et/ou ledit moyen de recueil sont inclus dans l'endoscope.

6. Dispositif selon l'une quelconque des revendications 1 à 5, caractérisé en ce que le marqueur fluorescent est avantageusement associé à des liposomes et/ou des anticorps monoclonaux appropriés.

7. Dispositif selon l'une quelconque des revendications 1 à 6, caractérisé en ce que le système de calcul du pH comprend avantageusement un moyen de calcul du rapport des signaux de fluorescence émise, obtenus successivement au moins auxdites deux longueurs d'ondes d'excitation, et un moyen de lecture du pH correspondant au rapport obtenu, sur une courbe d'étalonnage dudit marqueur, en fonction du pH.

8. Dispositif selon la revendication 7, caractérisé en ce que le système de calcul comprend également un système de contrôle du moyen de bascule.

9. Dispositif de contrôle de l'hyperthermie dans le traitement des tumeurs par hyperthermie, caractérisé en ce qu'il comprend un dispositif de mesure de pH selon l'une quelconque des revendications 1 à 8, associé à un moyen de chauffage approprié de ladite tumeur.

10. Dispositif selon la revendication 9, caractérisé en ce que ledit moyen de chauffage est associé à un moyen de focalisation de la chaleur produite, notamment une sonde, un cathéter ou une antenne extérieure.

11. Procédé d'utilisation du dispositif selon l'une quelconque des revendications 1 à 8, permettant d'obtenir les informations caractéristiques de l'évolution dans le temps du pH d'une cible appropriée, caractérisé en ce qu'il comprend :
- la mise en contact de la cible à analyser avec un marqueur fluorescent, présentant, au moins deux pics d'excitation et un pic d'émission et dont le spectre d'émission est dépendant du pH ;
- l'excitation de la cible ainsi traitée, successivement auxdites longueurs d'onde d'excitation dudit marqueur fluorescent ;
- la mesure de la fluorescence émise par ladite cible à analyser, successivement auxdites longueurs d'onde d'excitation ; et
- le calcul du pH de ladite cible à analyser à partir du rapport des signaux de fluorescence émis, obtenus successivement au moins auxdites deux longueurs d'onde d'excitation, par la lecture du pH correspondant au rapport obtenu, sur une courbe d'étalonnage dudit marqueur, en fonction du pH.

12. Procédé selon la revendication 11, caractérisé en ce que préalablement à la mise en contact de la cible à analyser avec le marqueur fluorescent, ladite cible est mise en contact avec un sucre, notamment du glucose.

13. Procédé selon la revendication 11 ou la revendication 12, caractérisé en ce que le marqueur fluorescent est notamment choisi dans le groupe qui comprend la fluorescéine, la fluorescéine conjuguée au dextran ou à une autre molécule inerte (DF), la 5 et/ou 6 carboxyfluorescéine (CF), le 2′,7′-bis(carboxyéthyl)-5 et/ou 6-carboxyfluorescéine (BCECF) et leurs esters, la pyramine (8-hydroxypyrène-1,3,6-trisulfonate), le 4-méthylumbelliferone ou 4-méthyl-7-hydroxycoumarine (4-MU), le 3,6 dicyanohydroquinone (DHPN), le SNARF-1 et le SNAF-2 (respectivement le semi-naphtorhodofluor et la semi-naphtofluorescéine).

14. Procédé selon la revendication 13, caractérisé en ce que ledit marqueur fluorescent est associé à des liposomes et/ou des anticorps monoclonaux appropriés.

## Patentansprüche

1. Vorrichtung zur Messung des pH-Werts einer Probe vom Typ umfassend eine Lichtquelle (101) mit der Eignung zur Auswahl von mindestens zwei Anregungswellenlängen der Fluoreszenz eines fluoreszierenden Markers, der auf der Probe fixiert ist und dessen Fluoreszenzemissionsspektrum pH-abhängig ist und ein Mittel zum Nachweis und zum Ablesen der emittierten Fluoreszenz (60, 70, 80; 61, 52, 53) dadurch **gekennzeichnet**, daß
* sie zur Messung des pH-Werts ohne direkten Kontakt mit der Probe:
- mindestens ein Überträgermittel (30, 30′) der Lichtquelle in die Probe (40, 40′),
- mindestens ein Mittel zur Aufnahme (50, 50′) der emittierten Fluoreszenz,
- ein System zur Berechnung des pH-Werts der Probe aus dem Verhältnis der emittierten Fluoreszenzsignale, die als Folge von mindestens zwei Anregungswellenlängen (90, 901) erhalten wurden,
umfaßt, und
* daß die Lichtquelle mit einem Mittel zum Umschalten (106, 106′) von einer Anregungswellenlänge in die andere verbunden ist.

2. Vorrichtung nach Anspruch 1, dadurch **gekennzeichnet**, daß das Überträgermittel der Lichtquelle mindestens eine optische Faser umfaßt.

3. Vorrichtung nach Anspruch 1 oder 2, dadurch **gekennzeichnet**, daß das Mittel zur Aufnahme der Fluoreszenz mindestens eine optische Faser umfaßt.

4. Vorrichtung nach einem der Ansprüche 1 bis 3, dadurch **gekennzeichnet**, daß das Überträgermittel und/oder das Mittel zur Aufnahme mit einem Endoskop (51) verbunden sind, welches an einen Bildverstärker (52) gekoppelt ist, der seinerseits an eine Videokamera (53) angeschlossen ist.

5. Vorrichtung nach Anspruch 4, dadurch **gekenn****zeichnet**, daß das Überträgermittel und/oder das Mittel zur Aufnahme in dem Endoskop eingeschlossen sind.

6. Vorrichtung nach einem der Ansprüche 1 bis 5, dadurch **gekennzeichnet**, daß der Fluoreszenzmarker vorteilhafterweise mit Liposomen und/oder geeigneten mono-clonalen Antikörpern assoziiert ist.

7. Vorrichtung nach einem der Ansprüche 1 bis 6, dadurch **gekennzeichnet**, daß das System zur Berechnung des pH-Werts vorteilhafterweise ein Mittel zur Berechnung des Verhältnisses der Signale der emittierten Fluoreszenz, die als Folge von mindestens zwei Anregungswellenlängen erhalten wurden, und ein Mittel zur Ablesung des pH-Werts entsprechend dem erhaltenen Verhältnis auf einer Eichkurve des Markers als Funktion des pH-Werts umfaßt.

8. Vorrichtung nach Anspruch 7, dadurch **gekennzeichnet**, daß das System zur Berechnung ebenso ein System zur Kontrolle des Mittels zur Umschaltung umfaßt.

9. Vorrichtung zur Kontrolle der Hyperthermie bei der Behandlung von Tumoren durch Hyperthermie dadurch **gekennzeichnet**, daß sie eine Vorrichtung zur Messung des pH-Werts nach einem der Ansprüche 1 bis 8 zusammen mit einem geeigneten Erhitzungsmittels für den Tumor umfaßt.

10. Vorrichtung nach Anspruch 9, dadurch **gekennzeichnet**, daß das Mittel zur Erhitzung mit einem Mittel zur Fokussierung der produzierten Wärme, insbesondere einer Sonde, einem Katheder oder einer Außenantenne verbunden ist.

11. Verfahren zur Verwendung der Vorrichtung nach einem der Ansprüche 1 bis 8 zum Erhalt charakteristischer Informationen bezüglich der zeitlichen Entwicklung des pH-Werts einer geeigneten Probe, dadurch **gekennzeichnet**, daß man
- die zu analysierende Probe mit einem Fluoreszenzmarker in Kontakt bringt, der mindestens zwei Anregungsgipfel und einen Emissionsgipfel besitzt und dessen Emissionsspektrum pH abhängig ist,
- man die so behandelte Probe mittels der Anregungswellenlängen des Fluoreszenzmarkers anregt,
- die durch die zu analysierende Probe als Folge der Anregungswellenlängen emittierte Fluoreszenz mißt und
- den pH-Wert der zu analysierenden Probe ausgehend von dem Verhältnis der Signale der emittierten Fluoreszenz, erhalten als Folge von mindestens zwei Anregungswellenlängen, durch Ablesen des pH-Werts entsprechend dem erhaltenen Verhältnis auf einer Eichkurve des Markers als Funktion des pH-Werts berechnet.

12. Verfahren nach Anspruch 11, dadurch **gekenn****zeichnet**, daß vor dem In-Kontakt-Bringen der zu analysierenden Probe mit dem Fluoreszenzmarker die Probe mit einem Zucker, insbesondere Glucose, in Kontakt gebracht wird.

13. Verfahren nach Anspruch 11 oder 12, dadurch **ge****kennzeichnet**, daß der Fluoreszenzmarker, insbesondere aus der Gruppe umfassend Fluorescein, an Dextran oder ein anderes inertes Molekül (DF) konjugiertes Fluorescein, 5-und/oder 6-Carboxyfluorescein (CF) 2′,7′-Bis-5- und/oder 6-(carboxyethyl)carboxyfluorescein (BCECF) und ihre Ester, Pyramin (8-Hydroxypyrrol-1,3,6-trisulfonat), 4-Methylumbelliferon oder 4-Methyl-7-hydroxycumarin (4-MU), 3,6-Dicyanohydrochinon (DHPN), 1-SNARF und 2-SNARF (jeweils Semi-Naphthorhodofluor und Semi-Naphthofluorescein) ausgewählt wird.

14. Verfahren nach Anspruch 13, dadurch **gekennzeichnet**, daß der Fluoreszenzmarker mit geeigneten Liposomen und/oder geeigneten monoclonalen Antikörpern assoziiert ist.

## Claims

1. Device for measuring the pH of a target of the type comprising a light source (101) appropriate for selecting at least two wavelenghts for excitation of the fluorescence of a fluorescent marker fixed to said target and whose fluorescence emission spectrum depends on the pH and a means for detecting and reading the emitted fluorescence (60, 70, 80; 61, 52, 53), said device being characterised
* in that it comprises, for measuring said pH without direct contact with said target:
- at least one transmission means (30, 30′) from said light source to the target (40, 40′);
- at least one means for collecting (50, 50′) the emitted fluorescence;
- a system for calculating the pH of the target from the ratio of the emitted fluorescence signals, obtained successively at least at said two excitation wavelenghts (90, 901), and
* in that the light source is associated with a means for switching (106, 106′) from one excitation wavelength to the other.

2. Device according to Claim 1, characterised in that the transmission means from the light source comprises at least one optical fibre.

3. Device according to Claim 1 or Claim 2, characterised in that the means for collecting fluorescence comprises at least one optical fibre.

4. Device according to any one of Claims 1 to 3, characterised in that said transmission means and/or said collector means are associated with an endoscope (51) coupled to an image intensifier (52) itself connected to a video camera (53).

5. Device according to Claim 4, characterised in that said transmission means and/or said collector means are enclosed in the endoscope.

6. Device according to any one of Claims 1 to 5, characterised in that the fluorescent marker is advantageously associated with appropriate liposomes and/or monoclonal antibodies.

7. Device according to any one of Claims 1 to 6, characterised in that the system for calculating the pH advantageously comprises means for calculating the ratio of the emitted fluorescence signals obtained successively at least at said two excitation wavelengths, and a means for reading the pH corresponding to the ratio obtained on a calibration curve of said marker as function of the pH.

8. Device according to Claim 7, characterised in that the calculating system also comprises a system for controlling the switching means.

9. Device for controlling hyperthermy in the treatment of tumours by hyperthermy, characterised in that it comprises a device for measuring pH according to any one of Claims 1 to 8, associated with an appropriate means for heating said tumour.

10. Device according to Claim 9, characterised in that said heating means is associated with a means for focusing the heat produced, especially a probe, a catheter or an external antenna.

11. Method for using the device according to any one of Claims 1 to 8, which makes it possible to obtain the data characteristic of the development over time of the pH of an appropriate target, characterised in that it comprises:
- bringing the target to be analysed into contact with a fluorescent marker having at least two excitation peaks and an emission peak and whose emission spectrum is dependent on the pH;
- successively exciting the target thus treated at said excitation wavelengths of said fluorescent marker;
- successively measuring the fluorescence emitted by said target to be analysed at said excitation wavelengths; and
- calculating the pH of said target to be analysed from the ratio of the emitted fluorescence signals, obtained successively at least at said two excitation wavelengths, by reading the pH corresponding to the ratio obtained on a calibration curve of said marker as a function of the pH.

12. Method according to Claim 11, characterised in that, prior to bringing the target to be analysed into contact with the fluorescent marker, said target is brought into contact with a sugar, especially glucose.

13. Method according to Claim 11 or Claim 12, characterised in that the fluorescent marker is especially chosen from the group which comprises fluorescein, fluorescein conjugated with dextran or with another inert molecule (DF), 5- and/or 6-carboxyfluorescein (CF), 2′,7′-bis(carboxyethyl)-5-and/or 6-carboxyfluorescein (BCECF) and their esters, pyramine (8-hydroxypyrene-1,3,6-trisulphonate), 4-methylumbelliferone or 4-methyl-7-hydroxycoumarin (4-MU), 3,6-dicyanohydroquinone (DHPN), SNARF-1 and SNAF-2 (respectively semi-naphthorhodofluor and semi-naphthofluorescein).

14. Method according to Claim 13, characterised in that said fluorescent marker is associated with appropriate liposomes and/or monoclonal antibodies.
